# EUROPEAN PATENT APPLICATION

(11) **EP 0 749 756 A2**
(43) Date of publication of application: **27.12.1996**
(21) Application number: 96304620.6
(22) Date of filing: 21.06.1996
(51) Int. Cl.: A61L 15/58

(54) **Adhesive film for adhesive bandage and adhesive bandage using said adhesive film**

(30) Priority: 22.06.1995 JP 177962/95
(71) Applicant: JOHNSON & JOHNSON CONSUMER PRODUCTS, INC., Skillman, New Jersey 08550 (US)
(72) Inventor: Saito, Toshikazu, Sukagawa-shi, Fukushima-ken (JP); Kumakura, Masahiro, Sukagawa-shi, Fukushima-ken (JP)
(74) Representative: Mercer, Christopher Paul

(57) **Abstract**

An adhesive film for adhesive bandage formed by coating an adhesive on one surface of a nonwoven fabric made of a polyester elastomer characterized in that tensile elongation at break of the nonwoven fabric is 600% or more and a weight thereof is 40g/m² or more and 200g/m² or less.

The adhesive bandage using the adhesive film of the present invention is, because utilizing the nonwoven fabric rich in flexibility and stretchability, excellent in fitting to a skin, does not disturb any skin movement and can prevent physical skin irritation. Further, because of the excellent water vapor permeability of the film of the present invention, skin respiration is not disturbed and diseases caused by stickiness with perspiration such as a rash and an eruption can be prevented.

## Description

This invention relates to an adhesive film for adhesive bandage and an adhesive bandage using said adhesive film. More specifically, the present invention relates to an adhesive film for adhesive bandage using as a backing a nonwoven fabric made of a polyester elastomer having high elasticity and water vapor permeability and an adhesive bandage using said adhesive film.

Conventionally, as an adhesive film for adhesive bandage, films formed by coating an adhesive on one surface of a plastic film made of such as polyvinylchloride, polyethylene and polypropylene are widely used. The materials such as polyvinyl chloride, polyethylene and polypropylene are, however, low in water vapor permeability; it tends to prevent skin respiration and cause skin irritation when an adhesive bandage whose backings are made of said materials are applied to a skin.

To prevent same, measures such as formation of air holes on the film have been carried out. However, it still locally prevents skin respiration. Further because the film is less flexible than the skin, it causes physical irritation to the skin. To improve water vapor permeability, films formed by coating a pressure-sensitive adhesive on one surface of nonwoven fabrics made of non-elastic fibers of such as rayons have been heretofore widely used.

Said nonwoven fabrics are low in flexibility, and when it is adhered to a skin and stretched together with the skin, the skin is drawn because of low flexibility of the nonwoven fabric, and it tends to cause physical irritation. Furthermore, once it is stretched it holds the length as it is stretched and would not return to its original length because of lack of elasticity and restoring force, thereby there is a problem such that it peels off from the skin because the nonwoven fabric cannot follow the movement of the skin and forms wrinkles. And owing to lack of elasticity and restoring force after stretched as mentioned above, there is another problem such as low fitting feeling when applied to the skin. To remove the skin irritation, for example, Japanese Laid-open Patent Application No. Hei 5-309128 discloses an adhesive film for adhesive bandage which has an adhesive layer on a nonwoven fabric of a laminate film made of polyester film whose thickness is 0.5 to 6 µm and a nonwoven fabric film of polyester whose weight is 5 to 20g/m². Japanese Laid-open Patent Application No. Hei 5-178741 discloses an adhesive film for adhesive bandage which is hard to peel off even with much perspiration and prevents a rash caused by such as stickiness by improving water absorbability using a fiber layer containing water-absorbing fiber. Further, Japanese Laid-open Patent Application No. Hei 5-138845 discloses a film or adhesive bandage made by laminating under heat a synthetic resin foam film and a nonwoven fabric of polyester. However the flexibility and fitting feeling of the adhesive films are not good enough for an adhesive bandage therefore the adhesive films are not well fit for an adhesive bandage.

Generally speaking, the smaller the modulus be, the more flexible and adjustable to the skin, especially when applied to a portion where large movement is indispensable, such as joints, for example, knees and elbows, high stretchability is necessary. there is not such a low modulus and high flexibility nonwoven fabric now commonly used that can suffice above mentioned demands to adhesive bandages. An object of the present invention is to provide, to solve above-mentioned problems, an adhesive film for adhesive bandage excellent in flexibility, elasticity, water vapor permeability and so forth.

This invention relates to an adhesive film for adhesive bandage formed by pattern-coating an pressure-sensitive adhesive or wholly coating or pattern-coating a porous adhesive having many thin holes on a polyester elastic fiber nonwoven fabric having excellent flexibility and elasticity, and to an adhesive bandage using said adhesive film.

The polyester elastomers constituting the nonwoven fabric used for an adhesive film in the present invention are elastomers whose base polymers are copolymers of polyalkylene terephthalate, constituting hard segments, and polymers of terephthalic acid esters and fatty polyethers, constituting soft segments. Examples of the polyalkylene terephthalate herein can be polyethylene terephthalate, polypropylene terephthalate, polybutylene terephthalate, etc. Especially polybutylene terephthalate is preferable. Examples of the fatty polyether can be polyethylene glycol, polypropylene glycol, polybutane diol, etc. Especially in order to obtain a flexible and high elastic nonwoven fabric, polybutane diol is preferable. A structural formula of the polyester elastomer of the present invention using polybutylene terephthalate and polybutane diol is as follows:

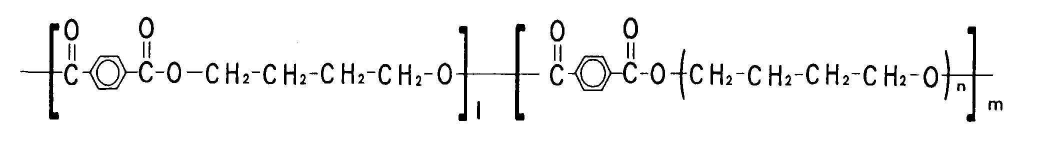

Wherein, 1 is a degree of polycondensation of 1,4-butane diol and terephthalic acid, m is degree of polymerization of polycondensation polymer of fatty polyether diol and terephthalic acid, n is degree of polycondensation of 1,4-butane diol.

Examples of the above polyester elastomer can be "GLYLAX E" (a trade name of Dainippon Ink and Chemicals Inc.), a block copolymer of polybutylene terephthalate and polyether. A method for manufacturing nonwoven fabrics from the above polymer is not particularly restricted and any conventional methods for manufacturing nonwoven fabrics are applicable, an example thereof is as follows: making polyester elastic filaments from the polyester elastomer by spinning by melt-blow method after heat-melting the above elastomer, multiplying said filaments without focussing, combining the filaments and fixing by heat-melting at contact points thereof.

Nonwoven fabrics of the present invention are excellent in heat resistance and chemical resistance from the inherent characteristics of the polyester elastomer (cf. "Elastomer" Shinzo Yamashita et al, Kyoritsu publishing), further it is necessary to possess flexibility, elasticity and tensile strength at break enough for adhesive films for adhesive bandages.

The flexibility can be expressed by modulus against elongation, but in the nonwoven fabric of the present invention, an excellent flexibility can be attained by rendering modulus at 5%, 10% and 100% elongation sufficiently small. The above modulus values of such nonwoven fabric excellent in flexibility, at 5%, 10% and 100% elongation are preferably, 4-80g/cm(0.01-0.2kg/inch), 16-120g/cm(0.04-0.3kg/inch) and 70-280g/cm(0.18-0.7kg/inch) respectively. More preferably,8-40g/cm(0.02-0.1kg/inch), 24-80g/cm(0.06-0.2kg/inch) and 80-200g/cm(0.2-0.5kg/inch) respectively.

Tensile elongation at break of the nonwoven fabric of the present invention is preferably 600% or more. Less than 600%, there are cases when a flexibility to a movement of the skin is not enough. Restoration ratio after repeated elongation is preferably 70% or more at repeated 100% elongation.

An adhesive force to the human skin is different largely among individuals and it is difficult to specify numerically, however, is approximately in the range from 100g/cm to 200g/cm. From this also, the tensile strength at break of the nonwoven fabric of the present invention is preferably 200g/cm(0.5kg/inch) or more in order to be suitable for the use of a backing film of an adhesive tape for adhesive bandage. Less than 200g/cm, the tensile strength at break is too small compared to adhesive force, and there are cases that the tape is broken when it is peeled off from the skin.

The thickness of the nonwoven fabric of the present invention is preferably 0.lmm or more, and preferably 0.4mm or less. More preferably 0.15mm or more and 0.3mm or less. Less than 0.lmm, stiffness of the nonwoven fabric is too low and it becomes hard to handle during application to the skin. More than 0.4mm, it is not preferable because a use feeling and a fitting feeling to a skin is bad when it is adhered to the skin.

A weight of the nonwoven fabric of the present invention is 40g/m² or more and 200g/m² or less. Preferably 50g/m² or more and 150g/m² or less. Less than 40g/m², the nonwoven fabric is so thin that the tensile strength at break is not enough, further it lacks the stiffness and it is difficult to handle during application to the skin. Furthermore the backing film thereof tends to break when the bandage is peeled from the skin. On the other hand, when it exceeds 200g/m², the use feeling becomes bad.

Adhesives used for the adhesive film for adhesive bandage of the present invention are not limited as far as they do not irritate the skin and have sufficient pressure-sensitive adhesion to the skin, and it can be easily peeled off from the skin after use. And rubber type adhesives, acrylic adhesives, polyurethane adhesives, silicone adhesives, styrene-isoprene-styrene type block copolymer adhesives can be used. Such adhesives can be coated on the whole adhesive surface of the nonwoven fabric, however to prevent decrease in the water vapor permeability, it is preferable to coat porous adhesives or to pattern-coat instead of wholly coating.

As a method to make adhesives porous, for example, a method can be taken which comprises using a high water absorbable polymer as a blowing agent, conducting water absorption, then dispersing it in an adhesive solution, coating the dispersion on a film surface, and evaporating the water to make the adhesive porous. However, said method is not critical.

Regarding the pattern coating, the adhesive can be coated on the backing film by, for example, screen coating method or gravure coating method. However, these coating methods are not critical.

As a method for coating an adhesive, there can be employed a method in which the adhesive is directly coated on the nonwoven fabric, a method in which the adhesive is coated on a release paper and then transferred onto the nonwoven fabric, and so forth.

The adhesive film for adhesive bandage of the present invention may be formed into the adhesive bandage by any method. Examples of the method include a method in which a long film having a suitable width is wound up to form an adhesive bandage, a method in which a film sheet of suitable size is provided, a water-absorbent pad is placed in the center of the sheet, and an adhesive surface is further covered with a protective sheet to form an individual package of an adhesive bandage, a method in which a sheet of suitable size is produced, and an adhesive surface is covered with a protective sheet without placing a pad to form an individual package of an adhesive bandage, and so forth.

The water-absorbent pad used here is preferably a pad made of cellulosic absorbent, polyester fiber absorbent and so forth, but not limited thereto as long as they have water absorbability. This invention will be illustrated more specifically by referring to the following Examples.

In Examples, properties were evaluated according to the following methods.
(1) Modulus:
   A sample was cut into strips having a width of 25.4 mm (1 inch), and each strip was mounted on a tensile tester at an interval of 50 mm. Tensile stresses were measured when the test pieces were stretched by 5 %, 10 % and 100 % at a pulling rate of 100 mm/min.
(2) Tensile strength at break and tensile elongation at break:
   A sample was cut to a width of 25.4 mm (1 inch), and each cut piece was mounted on a tensile tester at an interval of 50 mm. A pulling rate was set at 100mm/min., and tensile stress and elongation were measured when the test pieces were broken.
(3) Adhesion to glass:
   A sample strip was cut to a width of 25.4 mm (1 inch) and placed on a flat glass surface well washed with acetone and then dried. A weight load was exerted on the sample strip in order to adhere to the glass surface by one reciprocation of a roller made of an iron core having a weight of 4.5 kg wound with a rubber. The test sample was placed on the tensile tester, and a tensile stress was measured when the test strip was peeled from the glass surface at a pulling rate of 300 mm/min.
(4) Water vapor permeability:
   A sample film was adhered to a ring of a water vapor permeation cup according to JIS Z 0208, and surely mounted on a guide having a laboratory dish with water in it. This test sample was placed in a constant-temperature (32°C)/constant-humidity (30 RH%) room. A weight of the test sample was measured hourly until the weight difference per hour became stable. Water vapor permeability was calculated from the decrease in weight per hour.
(5) Thickness:
   A whole thickness in the state still adhesive surface was covered by a protective sheet was measured with a dial gage. Then the protective sheet was removed, the thickness of the protective sheet was measured with the dial gage, and the thickness of the adhesive film was calculated by subtracting the thickness of the protective sheet from the whole thickness.

### Example 1:

A block copolymer of polybutylene terephthalate-polyether (Trade name: GLYRAX E ; A product of Dainippon Ink and Chemicals, Inc.) was melt-kneaded with an extruder. The melt-kneaded copolymer was injected through a melt-blow spinning apparatus to form fibers. The fibers were collected, and pressure-bonded under heat to form a nonwoven fabric. A weight of the nonwoven fabric was 50 g/m². An acrylic adhesive dispersed particulates of highly water-absorbable polymer conducting water absorption as a blowing agent was coated on one surface of the nonwoven fabric such that the thickness of the adhesive become 50 µm, the adhesive on the nonwoven fabric was dried to render the adhesive porous, and an adhesive film for adhesive bandage was obtained. This adhesive film was used as samples and (1) modulus, (2) tensile strength at break and tensile elongation at break, (3) Adhesion to glass, (4) water vapor permeability and (5) thickness of the samples were measured.

### Example 2:

An adhesive film for adhesive bandage was obtained in the same manner as in Example 1 except that the weight of the nonwoven fabric was 75 g/m².

### Example 3:

A styrene-isoprene-styrene type block copolymer adhesive was pattern-coated on the same nonwoven fabric as used in Example 2, a fabric whose weight was 75g/m², so that the thickness of the adhesive was 50µm and above coating pattern of the adhesive was such that coated surface width are 2 mm and uncoated surface width was 2 mm. Thereby an adhesive film for adhesive bandage was obtained.

### Comparative Example 1:

Example 1 was repeated except that the weight of the nonwoven fabric was 30 g/m². And an adhesive film for adhesive bandage was obtained.

### Comparative Example 2:

Polypropyrene was added to a hydrogenated block copolymer of polystyrene-polyisoprene-polystyrene so that the content of the polypropyrene was 30 % by weight. The mixture was uniformly melt-kneaded and by melt-blow spinning method produced a nonwoven fabric "SEPT0N"(a trade name of Kuraray Co. Ltd.) In the same manner as in Example 1, the acrylic adhesive was coated on one surface of the nonwoven fabric such that the thickness became 50 µm to obtain an adhesive film for adhesive bandage.

### Comparative Example 3:

An acrylic adhesive dispersed particulates of highly water-absorbable polymer conducting water absorption as a blowing agent was coated on one surface of the polyurethane nonwoven fabric of 0.18 mm in thickness and 75g/m² in weight (a product of Kuraray Co. LTD.) so that the thickness of the adhesive was 50 µm, the coated film was dried to make the acrylic adhesive porous. Thereby an adhesive film for adhesive bandage was obtained. The adhesive films of above Examples and Comparative Examples were used as samples and the properties thereof were measured as in Example 1. The results were shown in Table 1.

### Table 1

**Table 1**

| Test item (unit) | Example No. | | | Conparative Ex. No. | | |
|---|---|---|---|---|---|---|
| | 1 | 2 | 3 | 1 | 2 | 3 |
| Modulus 5% (g/cm) | 16 | 16 | 20 | 4 | 39 | 16 |
| Modulus 10% (g/cm) | 28 | 32 | 39 | 16 | 79 | 32 |
| Modulus100% (g/cm) | 114 | 138 | 150 | 71 | 272 | 138 |
| Tensile strength at break (g/cm) | 236 | 350 | 335 | 122 | 555 | 390 |
| Tensile elongation at break (g/cm) | 693 | 674 | 657 | 361 | 511 | 476 |
| Adhesion to glass (g/cm) | >236 | 264 | >335 | >138 | 244 | 276 |
| Water vapor permeability(g/m ·24h) | 1130 | 1010 | 1110 | 1200 | 1590 | 1260 |
| Thickness (mm) | 0.18 | 0.20 | 0.20 | 0.10 | 0.20 | 0.18 |

From the results of Table 1, the adhesive film for adhesive bandage of the present invention is rich in flexibility and stretchability. The film is excellent in adjustability to a movement of the skin applied, and does not cause any physical skin irritation when actually adhered to a skin as an adhesive bandage. The tensile elongation at break of the adhesive film for adhesive bandage of the present invention, though it differs according to the weight of the nonwoven fabric, exceeds 600 % when the weight thereof is 75 g/m², which is higher than the value, about 450 %, of the polyurethane nonwoven fabric of the same weight. Also, the tensile strength at break is about 350 g/cm(about 0.9 kg/inch) which is sufficient for adhesive bandages. The water vapor permeability of human skin is generally said to be about 800 g/m²·24 hours, the adhesive films for adhesive bandage in any Examples of the present invention have more than the above value, which shows the film of the present invention has excellent water vapor permeability, consequently, the film does not prevent skin respiration and can prevent diseases caused by stickiness with perspiration. And when the adhesive film of any of the Examples was adhered actually to human skin for more than a day, no symptoms of a rash, an eruption and the like have been found.

As described hereinbefore, the adhesive film of the present invention has excellent properties as an adhesive film for adhesive bandage.

The adhesive bandage of the present invention is, owing to the nonwoven fabric rich in flexibility and stretchability, excellent in adjustability to the skin without disturbing skin movement. It can prevent physical skin irritation. Further, the film of the present invention does not disturb skin respiration and can prevent diseases caused by stickiness with perspiration such as rashes and eruptions because of the excellent water vapor permeability thereof.

## Claims

1. An adhesive film for adhesive bandage formed by coating an adhesive on one surface of a nonwoven fabric made of a polyester elastomer characterized in that tensile elongation at break of the nonwoven fabric is 600% or more and a weight thereof is 40g/m² or more and 200g/m² or less.

2. The adhesive film for adhesive bandage of above 1 wherein the polyester elastomer is polybutylene terephthalate-polyether copolymer.

3. The adhesive film for adhesive bandage of above 2 wherein a polyether potion of the polybutylene terephthalate-polyether copolymer is a polycondensation polymer of butanediol.

4. The adhesive film for adhesive bandage of any one of above 1 to 3 characterized in that the thickness of the nonwoven fabric is 0.1 mm or more and 0.4 mm or less.

5. The adhesive film for adhesive bandage of any one of above 1 to 3 characterized in that the thickness of the nonwoven fabric is 0.15 mm or more and 0.3 mm or less.

6. The adhesive film for adhesive bandage of any one of above 1 to 5 characterized in that the tensile modulus at 5% elongation, 10% elongation and 100% elongation of the nonwoven fabric are 8 to 40g/cm, 24 to 80g/cm, and 80 to 200g/cm respectively.

7. The adhesive film for adhesive bandage of any one of above 1 to 6 characterized in that the tensile strength at break is 200g/cm or more.

8. The adhesive film for adhesive bandage of any one of above 1 to 7 characterized in that the weight of the nonwoven fabric is 50g/m² or more and 150g/m² or less.

9. An adhesive bandage using the adhesive film of any one of above 1 to 8.
